# FASCICULE DE BREVET EUROPEEN

(11) **EP 1 830 760 B1**
(45) Date de publication et mention de la délivrance du brevet: **04.03.2009**
(21) Numéro de dépôt: 05850538.9
(22) Date de dépôt: 20.12.2005
(51) Int. Cl.: A61F 13/06, A61F 5/01, A43B 7/26

(54) **ECARTEUR ORTHOPEDIQUE ASSEMBLE**
MONTIERTER ORTHOPÄDISCHER RETRAKTOR
ASSEMBLED ORTHOPEDIC RETRACTOR

(30) Priorité: 28.12.2004 FR 0413973
(43) Date de publication de la demande: 12.09.2007
(73) Titulaire: Millet Innovation, 26270 Loriol sur Drome (FR)
(72) Inventeur: MILLET, Jean-Claude, F-26800 Etoile Sur Rhone (FR)
(74) Mandataire: Marchand, André
(86) Numéro de dépôt international: PCT/FR2005/003187
(87) Numéro de publication internationale: WO 2006/070098

(56) Documents cités:
- FR-A- 2 626 170
- US-A- 2 503 656
- US-A- 2 603 212
- US-A- 4 961 732

## Description

La présente invention concerne un écarteur orthopédique.

Les écarteurs orthopédiques sont couramment employés pour la correction de certains dérèglements du pied, par exemple un Hallux Valgus (déviation exagérée du gros orteil vers le dehors) ou pour prévenir et soulager les douleurs liées à un cor ou à un oeil de perdrix situé à la base des espaces interdigitaux. Ainsi, de tels écarteurs s'insèrent entre les orteils à soigner et permettent de séparer et d'écarter les orteils de quelques millimètres.

Les écarteurs orthopédiques sont généralement réalisés en gel silicone, par exemple un gel silicone commercialisé par la demanderesse sous l'appellation Epithélium26^{®}. Ils sont très souples, offrent un confort immédiat, restent bien en place durant la journée, sont lavables et réutilisables.

Les écarteurs ont généralement une forme qui est dictée par la recherche d'une bonne adaptation morphologique avec les orteils. Certains ont la forme d'une bille, d'autres ont la forme d'un tube, mais la forme généralement préférée est la forme appelée "diabolo".

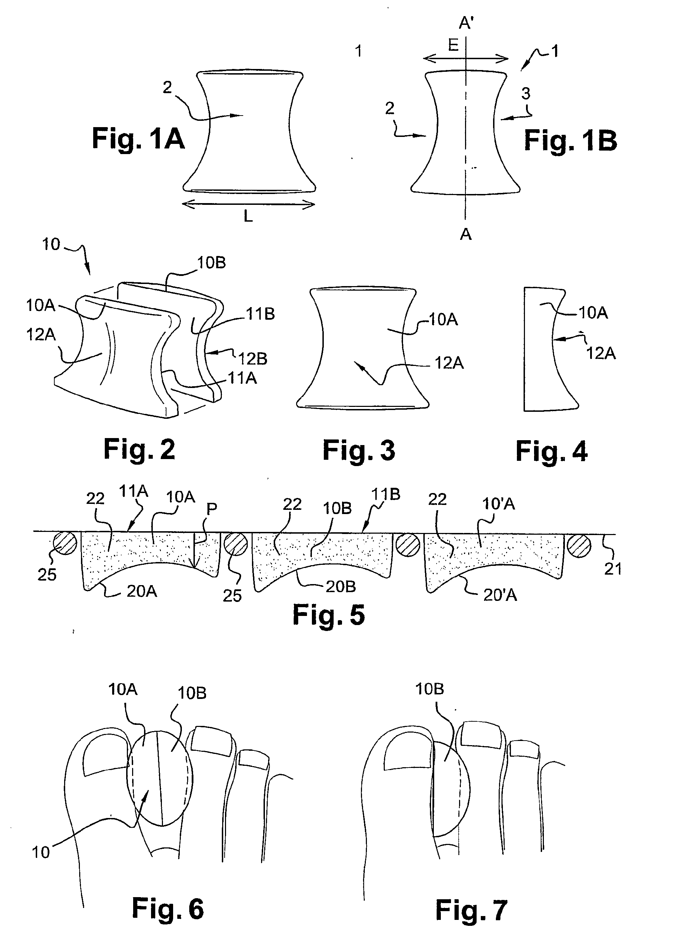

Un écarteur classique 1 de type diabolo est représenté respectivement par une vue de côté et une vue de profil sur les figures 1 et 2. On voit que la forme diabolo est sensiblement différente de celle du jeu de même nom. Elle s'apparente à celle d'un cylindre cintré, présentant à mi-hauteur un diamètre plus faible (mais non nul) qu'à ses deux extrémités, qui aurait ensuite été aplati par écrasement pour présenter une épaisseur E (vue de profil de la figure 1B) correspondant à l'écartement que l'on souhaite imposer aux orteils à soigner, et une longueur L (vue de côté de la figure 1A) inférieure à la longueur des orteils.

L'écarteur 1 est ainsi destiné à être agencé entre deux orteils dans le sens de son épaisseur E, et présente dans le sens de sa longueur deux faces latérales extérieures 2, 3 sensiblement concaves qui reçoivent les bords des orteils.

Un tel écarteur est généralement réalisé au moyen d'un moule, dans lequel le gel est introduit en phase liquide puis est réticulé (polymérisé) avant d'être démoulé. Ce procédé de fabrication est toutefois coûteux en raison du prix de revient du moule, des étapes d'introduction du gel non réticulé dans le moule et des étapes de démoulage. En particulier, le moule doit présenter deux parties qui sont jointives de façon étanche et qui sont ensuite séparées pour extraire l'écarteur du moule.

Un autre inconvénient d'un tel écarteur est que son épaisseur E ne convient pas nécessairement à toutes les morphologies de pieds et écartements d'orteils à soigner, de sorte qu'il doit être décliné en divers modèles, ce qui augmente encore plus son prix de revient.

La présente invention vise ainsi un écarteur qui soit simple à fabriquer et d'un prix de revient réduit, ainsi qu'un procédé de fabrication d'un tel écarteur.

La présente invention se fonde sur la constatation que les gels utilisés pour réaliser les écarteurs, et notamment les gels silicone, bien qu'étant moyennement adhésifs sur la peau, ont la particularité, lorsqu'on les met en contact, de former une jonction très résistante au cisaillement. Ainsi, si l'on accole deux surfaces planes en gel silicone réticulé, les surfaces adhèrent fortement en présence de forces de cisaillement (forces exercées parallèlement aux deux surfaces et qui pourraient les faire glisser l'une relativement à l'autre) mais adhèrent faiblement en arrachement, de sorte qu'elles restent facilement détachables.

L'invention se fonde sur cette propriété et prévoit de réaliser un écarteur comprenant deux pièces ou "demi-écarteurs" ayant chacune une face plane destinée à venir au contact de la surface plane correspondante de l'autre pièce, et formant ensemble l'équivalent d'un écarteur classique. Comme l'écarteur est destiné à être placé dans un espace interdigital, les deux pièces sont en outre soumises à une force perpendiculaire aux faces de contact, qui augmente la résistance de l'ensemble au cisaillement.

Plus particulièrement, la présente invention prévoit un écarteur orthopédique en gel polymère destiné à être inséré dans un espace interdigital, comportant deux parties complémentaires en gel polymère, chaque partie comprenant une face plate qui est accolée ou destinée à être accolée à la face plate de l'autre partie pour que les deux parties forment ensemble l'écarteur, les faces plates une fois accolées formant une jonction résistant à des forces de cisaillement tout en restant détachables.

Selon un mode de réalisation, les deux parties sont de formes identiques ou symétriques et de même épaisseur.

Selon un mode de réalisation, chaque partie est en gel silicone.

Selon un mode de réalisation, l'écarteur comprend un principe actif antibactérien ou anti-inflammatoire ou une combinaison des deux principes actifs.

La présente invention concerne également un procédé de fabrication d'un écarteur orthopédique comportant deux parties en gel polymère, chaque partie comprenant une face plate destinée à être accolée à la face plate de l'autre partie pour que les deux parties forment ensemble l'écarteur, les faces plates une fois accolées formant une jonction résistant à des forces de cisaillement tout en restant détachables, le procédé comprenant la prévision de deux moules ouverts, une étape d'insertion du gel polymère non réticulé dans chaque moule et une étape de réticulation du gel polymère dans chaque moule, les moules étant conformés de manière que la face ouverte de chaque moule corresponde à la face plate de chaque pièce.

Selon un mode de réalisation, les moules sont formés par déformation d'un matériau plastique.

Selon un mode de réalisation, les moules sont formés par thermoformage d'un matériau plastique.

Selon un mode de réalisation, le procédé comprend une étape de découpe des pourtours de chaque moule de manière que chaque moule forme un emballage de chaque pièce de l'écarteur, et une étape de conditionnement des pièces en vue de leur commercialisation, en les laissant dans leurs moules respectifs.

Selon un mode de réalisation, le procédé comprend une étape d'insertion dans le gel non réticulé d'un principe actif antibactérien ou anti-inflammatoire, ou d'un mélange des deux principes actifs.

Selon un mode de réalisation, les moules ont des formes identiques ou symétriques.

La présente invention concerne également un objet orthopédique en gel polymère destiné à être inséré dans un espace interdigital pour former un écarteur ou un séparateur, présentant une face plate destinée venir en appui sur une zone du pied à soigner ou à protéger et une face concave dont la fonction est de faciliter le maintien de l'objet dans l'espace interdigital.

Selon un mode de réalisation, l'objet est en gel silicone.

Selon un mode de réalisation, l'objet comprend un principe actif antibactérien ou anti-inflammatoire ou une combinaison des deux principes actifs.

La présente invention concerne également un procédé de fabrication d'un objet orthopédique en gel polymère destiné à être inséré dans un espace interdigital pour former un écarteur ou un séparateur, l'objet présentant une face plate destinée venir en appui sur une zone du pied à soigner ou à protéger et une face concave dont la fonction est de faciliter le maintien de l'objet dans l'espace interdigital, le procédé comprenant la prévision d'une moule ouvert, une étape d'insertion du gel polymère non réticulé dans le moule et une étape de réticulation du gel polymère dans le moule, le moule étant conformé de manière que la face ouverte du moule corresponde à la face plate de l'objet.

Selon un mode de réalisation, le moule est formé par déformation d'un matériau plastique.

Selon un mode de réalisation, le moule est formé par thermoformage d'un matériau plastique.

Selon un mode de réalisation, le procédé comprend une étape de découpe des pourtours du moule de manière que le moule forme un emballage de l'objet, et une étape de conditionnement de l'objet en vue de sa commercialisation, en le laissant à l'intérieur du moule.

Selon un mode de réalisation, le procédé comprend une étape d'insertion dans le gel non réticulé d'un principe actif antibactérien ou anti-inflammatoire, ou d'un mélange des deux principes actifs.

Ces objets, caractéristiques et avantages ainsi que d'autres de la présente invention seront exposés plus en détail dans la description suivante d'un écarteur orthopédique selon l'invention, faite à titre non limitatif en relation avec les figures jointes parmi lesquelles :
- les figures 1A et 1B sont des vues de côté et de profil d'un écarteur classique,
- la figure 2 est une vue éclatée d'un écarteur en deux parties selon l'invention,
- la figure 3 est une vue de côté d'une partie de l'écarteur selon l'invention,
- la figure 4 est une vue de profil d'une partie de l'écarteur selon l'invention,
- la figure 5 est une vue en coupe illustrant un procédé de fabrication d'un écarteur selon l'invention,
- la figure 6 illustre un exemple d'utilisation d'un écarteur selon l'invention, et
- la figure 7 illustre un exemple d'utilisation isolée d'une partie d'un écarteur selon l'invention.

Un écarteur 10 selon l'invention tel que représenté en figure 2 comprend deux parties complémentaires 10A, 10B qui comportent chacune une face plate 11A, 11B. Chaque partie 10A, 10B est réalisée en un gel polymère, de préférence un gel silicone, de sorte que les faces plates 11A, 11B adhèrent l'une à l'autre lorsqu'elles sont accolées. Comme exposé plus haut, cette adhérence typique des gels polymères se traduit par une excellente cohésion de l'ensemble en présence de forces de cisaillement qui tendent à faire glisser les surfaces 11A, 11B l'une relativement à l'autre, tandis que les surfaces 11A, 11B restent faiblement adhérentes en arrachement et peuvent être séparées après avoir été mises en contact. Or, la cohésion des deux parties face à des forces de cisaillement est suffisante pour satisfaire l'application visée car, lorsque l'écarteur 10 se trouve agencé dans un espace interdigital, les orteils maintiennent les deux parties 10A, 10B entre elles et celles-ci ne subissent essentiellement que des forces de cisaillement.

Le gel polymère ou silicone est choisi de manière que ses propriétés soient voisines de celles des tissus humains du pied. Il s'agit par exemple de l'EPITHELIUM 26^{®} cité plus haut ou d'un matériau équivalent. Dans ces conditions, l'utilisateur ne ressent plus la présence de l'écarteur dès que l'adaptation proprioceptive est intervenue, soit après quelques heures d'utilisation.

La forme représentée sur la figure 2 est celle d'un écarteur diabolo, qui est la plus couramment employée, mais l'invention s'applique à toute forme d'écarteur. Ainsi, chaque partie 10A, 10B présente à l'opposé des faces plates 11A, 11N, des faces extérieures 12A, 12B sensiblement convexes correspondant aux faces externes 2, 3 de l'écarteur classique 1 représenté sur la figure 1B et précédemment décrit.

La figure 3 représenté la pièce 10A vue du côté de sa face externe 12A. Vue sous cet angle, celle-ci ne se distingue pas de l'écarteur classique 1. Par contre, sur la figure 4 qui représente la pièce 10A vue de profil, il apparaît que celle-ci est l'équivalent d'une moitié de l'écarteur classique 1 qui aurait été obtenue en coupant l'écarteur 1 selon un plan longitudinal AA' passant par le milieu de l'écarteur 1.

Toutefois, et de préférence, les pièces 10A, 10B formant l'écarteur 10 ne sont pas obtenues en coupant en deux parties un écarteur classique mais en réalisant les deux parties au moyen de deux moules distincts, ainsi que cela va maintenant être décrit.

La figure 5 illustre un mode de réalisation préféré de l'écarteur 10 selon l'invention. Des moules ouverts 20A, 20B sont réalisés en imprimant des empreintes creuses dans une fine feuille de matière plastique 21. Ces empreintes correspondent à la forme extérieure des parties 10A, 10B et notamment à la forme des faces extérieures 12A, 12B des parties 10A, 10B. Elles sont de préférence réalisées par thermoformage (déformation à chaud) en utilisant une matière plastique appropriée, par exemple un film en polyéthylène ou en PVC.

Les moules 20A, 20B peuvent être de même forme si l'écarteur 10 présente une symétrie relativement à un plan perpendiculaire aux faces 11A, 11B passant par le centre de l'écarteur (ce qui est le cas en figure 2, les deux moules étant ici identiques). Dans le cas contraire, si l'écarteur ne présente qu'une une symétrie relativement aux faces 11A, 11B elles sont de formes symétriques et complémentaires (formant chacune le miroir de l'autre). De façon générale, il est préférable que les pièces 10A, 10B soient de même épaisseur, de sorte que les moules ont de préférence la même profondeur, c'est-à-dire une même profondeur P en des points similaires puisque le fond des moules n'est pas plat en raison de la forme convexe des faces extérieures 12A, 12B.

Une fois les moules 20A, 20B réalisés, l'ensemble est agencé sur un support, par exemple un support grillagé comportant des barres horizontales 25 de soutien de la feuille plastique 21, les barres 25 passant entre les moules 20A, 20B. Un tel procédé se prête bien à une fabrication collective d'écarteurs selon l'invention et divers autres moules 20'A (un seul étant représenté) peuvent être avantageusement réalisés simultanément sur la feuille de plastique 21.

Une préparation de gel silicone 22 liquide non réticulé est ensuite introduit dans chacun des moules, en remplissant les moules jusqu'à un niveau déterminé, à ras bord sur la figure 5. Cette préparation comprend avantageusement un principe actif anti-bactérien ou anti-inflammatoire, de manière à soulager par diffusion transdermique des douleurs liées à des dérèglements osseux ou articulaires.

Le gel liquide s'étale de lui-même sous l'effet de la gravité, de sorte que les faces planes 11A 11B sont obtenues après réticulation sans qu'il soit nécessaire de prévoir une étape de découpe ou de reprise des pièces réalisées.

Dans un mode de réalisation avantageux du procédé, les moules 20A, 20B sont ensuite découpés sans démouler les pièces 10A, 10B, qui sont alors conditionnées avec leurs moules dans des emballages de transport, éventuellement après avoir déposé sur chaque moule une mince feuille protectrice (non représentée). Une feuille protectrice de plus grande taille couvrant l'ensemble des moules peut également être soudée ou collée sur les bords des moules avant l'étape de découpe et être découpée avec les moules pour obtenir une pluralité de feuilles de protectrices individuelles.

La figure 6 illustre une utilisation de l'écarteur 10 selon l'invention et représente l'écarteur 10 agencé entre le gros orteil et le second orteil. Comme indiqué plus haut, la force de compression exercée par les deux orteils maintient les parties 10A, 10B en place et les forces de cisaillement sont neutralisées par l'effet naturel d'adhérence entre les deux parties.

Avantageusement, et comme illustré sur la figure 7, l'une des deux parties 10A ou 10B peut également être utilisée seule comme séparateur d'orteils (élément de séparation sans fonction d'écartement) ou comme écarteur pour les pieds de petite taille. L'homme de l'art notera ainsi que chaque partie 10A ou 10B selon l'invention - séparateur ou écarteur selon la taille du pied - forme en elle-même un objet selon l'invention. Un tel objet se distingue d'un séparateur ou d'un écarteur classique en ce qu'il comporte une face plate permettant d'obtenir la meilleure répartition de charge sur la zone du pied à protéger ou à soigner, et une face concave ayant une fonction de maintien de l'objet dans le sens vertical.

Des objets 10A, 10B selon l'invention, de tailles et d'épaisseurs différentes, peuvent également être combinés pour correspondre à diverses tailles de pieds, et/ou pour obtenir des valeurs d'écartement résultant de la combinaison de leurs dimensions en accolant par exemple des objets 10A, 10B ayant des épaisseurs différentes.

## Revendications

1. Écarteur orthopédique (10) en gel polymère destiné à être inséré dans un espace interdigital, **caractérisé en ce qu'**il comporte deux parties (10A, 10B) complémentaires en gel polymère, chaque partie comprenant une face plate (11A, 11B) qui est accolée ou destinée à être accolée à la face plate de l'autre partie pour que les deux parties forment ensemble l'écarteur (10), les faces plates (11A, 11B) une fois accolées formant une jonction résistant à des forces de cisaillement tout en restant détachables.

2. Écarteur selon la revendication 1, dans lequel les deux parties (10A, 10B) sont de formes identiques ou symétriques et de même épaisseur.

3. Écarteur selon l'une des revendications 1 et 2, dans lequel chaque partie (10A, 10B) est en gel silicone.

4. Écarteur selon l'une des revendications 1 à 3, comprenant un principe actif antibactérien ou anti-inflammatoire ou une combinaison des deux principes actifs.

5. Procédé de fabrication d'un écarteur orthopédique (10) comportant deux parties (10A, 10B) en gel polymère, chaque partie comprenant une face plate (11A, 11B) destinée à être accolée à la face plate de l'autre partie pour que les deux parties forment ensemble l'écarteur, les faces plates une fois accolées formant une jonction résistant à des forces de cisaillement tout en restant détachables, procédé comprenant la prévision de deux moules ouverts (20A, 20B), une étape d'insertion du gel polymère non réticulé dans chaque moule et une étape de réticulation du gel polymère dans chaque moule, les moules étant conformés de manière que la face ouverte de chaque moule corresponde à la face plate (11A, 11B) de chaque pièce.

6. Procédé selon la revendication 5, dans lequel les moules sont formés par déformation d'un matériau plastique (21) .

7. Procédé selon la revendication 6, dans lequel les moules sont formés par thermoformage d'un matériau plastique (21).

8. Procédé selon l'une des revendications 6 et 7, comprenant une étape de découpe des pourtours de chaque moule (20A, 20B) de manière que chaque moule forme un emballage de chaque pièce (10A, 10B) de l'écarteur (10), et une étape de conditionnement des pièces en vue de leur commercialisation, en les laissant dans leurs moules respectifs.

9. Procédé selon l'une des revendications 5 à 8, comprenant une étape d'insertion dans le gel non réticulé d'un principe actif antibactérien ou anti-inflammatoire, ou d'un mélange des deux principes actifs.

10. Procédé selon l'une des revendications 5 à 9, dans lequel les moules (20A, 20B) ont des formes identiques ou symétriques.

## Claims

1. An orthopedic retractor (10) made of polymer gel intended to be inserted into an interdigital space, **characterized in that** it comprises two complementary parts (10A, 10B) made of polymer gel, each part comprising a flat face (11A, 11B) which is assembled or intended to be assembled to the flat face of the other part so that together the two parts form the retractor (10), the flat faces (11A, 11B) once assembled forming a junction resistant to shearing forces while remaining removable.

2. Retractor according to claim 1, wherein the two parts (10A, 10B) are identical or symmetrical in shape and of the same thickness.

3. Retractor according to one of claims 1 and 2, wherein each part (10A, 10B) is made of silicone gel.

4. Retractor according to one of claims 1 to 3, comprising an active antibacterial or anti-inflammatory substance or a combination of the two active substances.

5. A method for manufacturing an orthopedic retractor (10) comprising two parts (10A, 10B) made of polymer gel, each part comprising a flat face (11A, 11B) intended to be assembled to the flat face of the other part so that together the two parts form the retractor, the flat faces once assembled forming a junction resistant to shearing forces while remaining removable, the method comprising providing two open molds (20A, 20B), a step of inserting the non-cross-linked polymer gel into each mold and a step of cross-linking the polymer gel in each mold, the molds being formed so that the open face of each mold corresponds to the flat face (11A, 11B) of each part.

6. Method according to claim 5, wherein the molds are formed by deformation of a plastic material (21).

7. Method according to claim 6, wherein the molds are formed by thermoforming a plastic material (21).

8. Method according to one of claims 6 and 7, comprising a step of cutting the perimeters of each mold (20A, 20B) so that each mold forms a package for each piece (10A, 10B) of the retractor (10), and a step of packaging the pieces in order to market them, by leaving them in their respective molds.

9. Method according to one of claims 5 to 8, comprising a step of inserting an active antibacterial or anti - inflammatory substance, or a combination of the two active substances, into the non-cross-linked gel.

10. Method according to one of claims 5 to 9, wherein the molds (20A, 20B) are identical or symmetrical in shape.

## Patentansprüche

1. Orthopädische Spreizvorrichtung (10) aus Polymergel, die dazu bestimmt ist, in einen Raum zwischen den Zehen eingefügt zu werden, **dadurch gekennzeichnet, dass** sie zwei komplementäre Teile (10A, 10B) aus Polymergel aufweist, wobei jeder Teil eine flache Seite (11A, 11B) aufweist, die an die flache Seite des anderen Teils angelegt ist oder dazu bestimmt ist, an sie angelegt zu werden, damit die zwei Teile die Spreizeinheit (10) bilden, wobei die flachen Seiten (11A, 11B), sobald sie aneinander angelegt sind, eine Verbindung bilden, die Scherkräften widersteht und gleichzeitig abnehmbar bleiben.

2. Spreizvorrichtung nach Anspruch 1, bei der die zwei Teile (10A, 10B) mit identischer oder symmetrischer Form und mit der gleichen Stärke ausgebildet sind.

3. Spreizvorrichtung nach einem der Ansprüche 1 und 2, bei der jeder Teil (10A, 10B) aus Silikongel besteht.

4. Spreizvorrichtung nach einem der Ansprüche 1 bis 3, die einen antibakteriellen oder entzündungshemmenden Wirkstoff oder eine Kombination der zwei Wirkstoffe enthält.

5. Verfahren zum Herstellen einer orthopädischen Spreizvorrichtung (10), die zwei Teile (10A, 10B) aus Polymergel aufweist, wobei jeder Teil eine flache Seite (11A, 11B) aufweist, die dazu bestimmt ist, an die flache Seite des anderen Teils angelegt zu werden, damit die zwei Teile gemeinsam die Spreizvorrichtung bilden, wobei die aneinander gelegten flachen Seiten eine Verbindung bilden, die Scherkräften standhält und gleichzeitig abnehmbar bleiben, wobei das Verfahren das Vorsehen von zwei offenen Formen (20A, 20B), einen Schritt des Einfügens des nicht vernetzten Polymergels in jede Form und einen Schritt des Vernetzens des Polymergels in jeder Form aufweist, wobei die Formen derart ausgebildet sind, dass die offene Seite jeder Form der flachen Seite (11A, 11B) jedes Teils entspricht.

6. Verfahren nach Anspruch 5, bei dem die Formen durch Verformen eines Kunststoffs (21) gebildet sind.

7. Verfahren nach Anspruch 6, bei dem die Formen durch Wärmeformen eines Kunststoffs (21) gebildet werden.

8. Verfahren nach einem der Ansprüche 6 und 7, das einen Schritt des Ausschneidens der Konturen jeder Form (20A, 20B) derart aufweist, dass jede Form eine Verpackung jedes Teils (10A, 10B) der Spreizvorrichtung (10) bildet, und einen Schritt des Verpackens der Teile zu ihrer Vermarktung, indem sie in ihren jeweiligen Formen gelassen werden.

9. Verfahren nach einem der Ansprüche 5 bis 8, das einen Schritt des Einfügens in das nicht vernetzte Gel eines antibakteriellen oder entzündungshemmenden Wirkstoffs oder eines Gemischs der zwei Wirkstoffe aufweist.

10. Verfahren nach einem der Ansprüche 5 bis 9, bei dem die Formen (20A, 20B) identische oder symmetrische Formen haben.
